# EUROPÄISCHE PATENTSCHRIFT

(11) **EP 1 902 723 B1**
(45) Veröffentlichungstag und Bekanntmachung des Hinweises auf die Patenterteilung: **08.07.2009**
(21) Anmeldenummer: 06019583.1
(22) Anmeldetag: 19.09.2006
(51) Int. Cl.: A61K 36/81, A61K 36/8962, A61K 36/889, A61P 3/00

(54) **Sabalfrüchte oder Sabalöl als Lipaseinhibitor**
Sabal fruit or Sabal oil as lipase inhibitor
Fruit ou huile de Sabal comme inhibiteur de lipase

(43) Veröffentlichungstag der Anmeldung: 26.03.2008
(73) Patentinhaber: Schrezenmeir, Jürgen, Prof. Dr., 24114 Kiel (DE)
(72) Erfinder: Schrezenmeir, Jürgen, Prof. Dr., 24114 Kiel (DE)
(74) Vertreter: Pöhner, Wilfried Anton, Dr.

(56) Entgegenhaltungen:
- WO-A-97/33599
- WO-A-20/06097074
- DE-A1- 3 520 761
- DE-A1- 4 101 841
- DE-A1- 10 041 403
- US-B1- 6 174 542
- US-B1- 6 319 524
- US-B1- 6 348 502
- AGARWAL K C: "THERAPEUTIC ACTIONS OF GARLIC CONSTITUENTS" MEDICINAL RESEARCH REVIEWS, NEW YORK, NY, US, Bd. 16, Nr. 1, 1996, Seiten 111-124, XP002917171 ISSN: 0198-6325
- EL-DEMERDASH F M ET AL: "Biochemical study on the hypoglycemic effects of onion and garlic in alloxan-induced diabetic rats" FOOD AND CHEMICAL TOXICOLOGY, XX, XX, Bd. 43, Nr. 1, Januar 2005 (2005-01), Seiten 57-63, XP004668597 ISSN: 0278-6915
- TOLAN IAN ET AL: "Isolation and purification of the hypoglycaemic principle present in Capsicum frutescens." PHYTOTHERAPY RESEARCH : PTR JAN 2004, Bd. 18, Nr. 1, Januar 2004 (2004-01), Seiten 95-96, XP009076512 ISSN: 0951-418X
- RAO R RAMAKRISHNA ET AL: "In vitro influence of spices and spice-active principles on digestive enzymes of rat pancreas and small intestine." NAHRUNG, Bd. 47, Nr. 6, Dezember 2003 (2003-12), Seiten 408-412, XP009076496 ISSN: 0027-769X
- DATABASE WPI Week 199744 Derwent Publications Ltd., London, GB; AN 1997-471649 XP002416378 & CN 1 119 498 A (SANHE IND CO LTD QIYANG COUNTY HUNAN PRO) 3. April 1996 (1996-04-03)
- DATABASE WPI Week 200545 Derwent Publications Ltd., London, GB; AN 2005-445060 XP002416379 & WO 2005/056031 A1 (JAPAN INT RES CENT AGRIC SCI MIN AGRIC) 23. Juni 2005 (2005-06-23)

## Beschreibung

Die Erfindung bezieht sich auf die Verwendung von Sabalfrüchten. (Sabalae serulatae) als gesamte Frucht oder mit dem durch Pressen der Frucht hergestellten Öl als Einzelkomponente oder als Gemisch zur Herstellung eines oral zu applizierenden Arzneimittels oder eines funktionellen Lebensmittels oder eines Wirkstoffes in Lebensmitteln.

Fett kann durch exzessive Aufnahme zahlreiche Erkrankungen verursachen, wie z. B. Fettleibigkeit (Adipositas), hohe Blutfette (Hyperlipämie) und Typ 2 Diabetes als zentrale Erscheinungen des metabolischen Syndroms. Erhöhte Triglycerid- und Glukosewerte im Blut nach der Nahrungsaufnahme sind ein unabhängiger Risikofaktor für eine Insulinresistenz, die eine zentrale Bedeutung in der Entwicklung des metabolischen Syndroms darstellt. Eine Möglichkeit der Reduzierung des postprandialen Triglyceridspiegels ist die Senkung der Aktivität der Enzyme, die das Nahrungsfett hydrolysieren. Die Bauchspeicheldrüsenlipase ist wesentlich für den Abbau und die Absorption der durch Nahrungsaufnahme zugeführten Fette im Intestinaltrakt. Aus diesem Grunde führt deren Verringerung zu einer Vermeidung oder zumindest einer Verzögerung der intestinalen Verdauung der Nahrungsfette und demzufolge zu einer Verringerung der Bluttriglyceride.

Die Fettleibigkeit ist ein wichtiger Risikofaktor für den Typ-2-Diabetes. Es ist allgemein anerkannt, dass eine Gewichtsreduktion die Blutzuckerkontrolle verbessert und zu einer Reduzierung des Risikofaktors für kardiovaskuläre Erkrankungen führt. Im Allgemeinen wir es als schwierig angesehen, eine Gewichtsreduzierung zu erreichen und das verringerte Gewicht beizubehalten. Tetrahydrolipstatin (THL, Orlistat, Xenical) ist ein käuflich erwerbbarer Lipaseinhibitor, der aus Streptomyces toxystricini gewonnen wird. Nach oraler Verabreichung verhindert Orlistat den Fettabbau, indem die aktiven Abschnitte der pankreatischen Lipase (Bauchspeicheldrüse -Lipase) blockiert werden. In klinischen Versuchen führte Orlistat zu einer Gewichtsreduktion und zu einer Verbesserung des postprandialen (nach Nahrungsaufnahme) Fettprofiles (reduziert wurden die postprandialen Triglyceride, sowie das LDL-Cholesterin und das Gesamtcholesterin sowie das Verhältnis LDL zu HDL verbessert). Die Anwendung von Orlistat führt zu unerwünschten Nebeneffekten wie öligem und fettem Stuhl. Eine sanftere Lipasehemmung wird durch Anwendung von pflanzlichen Extrakten und deren aktiven Wirkstoffen erreicht, die in einer zeitliche Verzögerung anstelle einer kompletten Unterbindung der Fettabsorption resultieren und auf diese Weise öligen oder fetten Stuhl vermeiden können.

Die Verabreichung von Lipaseinhibtoren wird als nützliche Medikamion für Patienten mit Symptomen des metabolischen Syndroms erachtet. Zusätzlich können ein lipasehemmender Pflanzenextrakt oder hiervon isolierte Bestandteile als Nahrungszusätze verwendet werden.

Aus mehreren Patenten ist die Anwendung von Pflanzenextrakten als mögliche Inhibitoren für die Lipase im Verdauungstrakt genannt:

Nur beispielhaft seien die WO 2005056031, die WO 2005099735 und die WO 2005077384 aufgeführt.

In mehreren Veröffentlichungen wurden die positiven Effekte von Pflanzenextrakten auf das postprandiale Lipidprofil beschrieben, nämlich um folgende Substanzen wie Zyclokaria Paliurus, Weintraubenkernen, Salbei, Cassia mimosoides L. var., Nomame Makino und Alpinia officinarum.

Darüber hinausgehend hat sich die Erfindung die Ermittlung einer weiteren Pflanze zur Aufgabe gemacht, die als Lipase-Inhibitor einsetzbar ist. Dabei soll dieser Wirkstoff sowohl in Arzneimitteln wie auch als Lebensmittel oder als Bestandteil von funktionellen Lebensmitteln verwendbar sein und sowohl zur Therapie wie auch zur Prävention geeignet sein.

Zur Lösung dieser Aufgabe werden Sabalfrüchte *(Sabalae serulatae)* als gesamte Frucht oder als Extrakt sowie deren Mischung mit anderen, ebenfalls als Lipase-Inhibitor wirkenden Stoffen oder mit sonstigen Stoffen genannt. Ihr Vorteil ist, dass sie Bestandteil eines Arzneimittels sein können und/oder als Zusatz zu Lebensmitteln, und hier insbesondere zu funktionellen Lebensmitteln, einsetzbar sind.

Gemäß üblicher Terminologie enthält ein funktionelles Lebensmittel einen Bestandteil, der wissenschaftlich nachweisbar entweder erheblich und positiv auf die organischen Funktionen einwirkt oder das Risiko bestimmter Erkrankungen reduziert.

Dabei dienen sie zur Behandlung von bereits eingetretenen, nachteiligen Effekte eines überhöhten Verzehrs von Fetten, wie zum Beispiel der Diabetes.
Zusätzlich reduzieren die Wirkstoffe auch das Risiko für das Eintreten oder das Verstärken von den vorgenannten oder von ähnlichen, nachteiligen Wirkungen oder Erkrankungen.

Im Folgenden werden die Versuchsreihen beschrieben, mit welchen die beanspruchten Wirkungen plausibilisiert werden:

### Beschreibung der Herstellung der Extrakte

Die Extrakte aus Sabalfrüchten wurden durch Pressen zur Gewinnung des Pflanzenöls hergestellt.

### Beschreibung der Aufbereitung und der Verfahren zur Bestimmung der Enzymaktivitäten.

### Vorbereitung der Testlösung

Zur Vorbereitung der Testlösung aus extrahiertem Pulver und flüssigem Material, werden 200 mg des extrahierten Materials in 1 ml eines geeigneten Lösungsmittels aufgelöst und intensiv verrührt. Nach Zentrifugieren bei 3400 Umdrehungen für 5 Minuten wird der erhaltene flüssige Bestandteil als Testlösung genutzt. Zum Anpassen des Verhältnisses zwischen der Konzentration der festen Bestandteile und der Enzymmasse im letzten Versuch wurde die Testlösung im Verhältnis 1:200 verdünnt um die alkalische Phospatase zu bestimmen.

Zur Zubereitung der Testlösung aus CO₂-Auszügen und Ölharzen werden 100 µl öligen Flüssigkeitsextrakt in 900 µl eines geeigneten Enzympuffers gelöst und mit Ultraschall bei 100 Watt für 5 Minuten behandelt.

### Pankreatische Lipase (Bauchspeicheidrüsenlipase)

Zwei Lipasebestimmungen wurden durchgeführt um die Aktivität der pankreatischen Lipase in vitro zu ermitteln:
1. Das Assay für die Bestimmung der Lipaseaktivität besteht aus
   - 200 µl von einem 50 mM/L BICIN-Puffer (pH 8,0), der 1 % technisches Gummi arabicum enthält.
   - 50 µl Pflanzenextraktlösung und
   - 25 µl einer 0,35 mM/L pankreatischen Lipaselösung
      Diese Mischung wurde für 20 Minuten bei 37° inkubiert.
      Die letzte Messung der pankreatischen Lipaseaktivität wird einem Autoanalyser für klinische Chemie (Konelab, Kone) durchgeführt, unter Verwendung eines Lipasekolorimetertestes mit einer Diacylglycerol mit Metylresorufin in dritter Position als Substrat und Colipase und Gallensalze als wesentliche Co-Faktoren. Dieses Verfahren ist für die prankreatische Lipase spezifisch und basiert auf der Spaltung des Methylresorufin vom Substrat durch die Enzyme.
   Zur Bestimmung der pankreatischen Lipaseaktivität im Autoanalyzer wurde folgendes Pipettierschema gewählt:
   1. 60 µl Reagenz 1: Colipase und Cholat
   2. 2 µl Testlösung + 10 µl Waschlösung
   3. 180 Sekunden Inkubation
   4. 40 µl Reagenz 2: Colorimetrisches Substrat und Cholat
   5. 120 Sekunden Inkubation
   Das Methylresorufin wurde fotometrisch bei 75 Nanometern gemessen.
2. Die Lipaseinhibition unter dem Einfluss von Pflanzenextrakten wurde mit einer zweiten Lipaseaktivitätsmessmethode bestimmt, indem die Freisetzung der freien Fettsäure während der Hydrolyse aus Triolein durch das Enzym gemessen wird.
   Um das Inkubationsgemisch herzustellen, wird ein gebrauchsfertige Lösung (Reagenz R1) verwendet. R1 besteht aus:
   26 mmol/L Tris Puffer, pH 9,2
   19 mmol/L Natriumdesoxycholat
   0,1 1 mmol/L Calciumchlorid
   0,3 mmol/L Triolein
   300 KU/L Colipase
   Die Inkubationsmischung setzt sich wie folgt zusammen:
   50 µL 26 mmol/L Tris Puffer, pH 9,2
   50 µL 9,7 mmol/L Triolein Emulsion in R1
   50 µL 0.134 mol/L pancreatische Lipase in 26 mmol/L Tris Puffer pH 9,2
   50 µL Testlösung
   1 % Methylzellulose in 26 mmol/L Tris buffer (pH 9.2) wurde bei der Untersuchung der CO₂ Auszüge benutzt.

Nach einer Inkubationszeit von 30 Minuten bei 37° in einem Wasserbad wurde die Reaktion durch Beigabe von 3 ml einer Chloroform-Heptan-Methanol-Mischung (50:49:1) gestoppt. Die freigesetzten freien Fettsäuren wurden aus dem Reaktionsgemisch durch intensives Rühren für 10 Minuten extrahiert. Nach Zentrifugieren (3400 Umdrehungen, 10 Minuten) wird ein 2 ml Rest werden aus der Chloroformphase gewonnen und 1 ml aus einem Kupferreagenz (94 ml reines Wasser, 6ml 1 N NaOH, 6,45 mmol/L pro Cu(NO₃)₂, 0,1 mol/L Triethanolamin, 33 % (w/v) NaCl) wird zugegeben. Diese Mischung wurde intensiv für 10 Minuten gerührt und bei 3400 Umdrehungen für 10 Minuten zentrifugiert. Aus dem flüssigen Überstand wurden 1 ml entnommen und ein 1 ml Chloroform bei 0,1 % Bathocuproin und 0,05 (w/v) 3-Tert-butyl-4-hydroxyanisol wurden hinzugegeben. Der fette Säure- Kupfer- Bathocuproin-Komplex wird schließlich fotometrisch bei 480 nm in einem Standardfotometer bestimmt.

Um sicherzustellen, dass der Rückgang der Lipaseaktivität nicht auf einen unspezifisches Enzymhemmer bzw. Denaturierung des Enzymkomplexes zurückzuführen ist, wurden die Aktivität der α-Amylase und der alkalischen Phosphatase zusätzlich bestimmt. Eine Hemmung der α-Amylase kann sich als eine Verringerung des postprandialen Blutzuckerspiegels auswirken und wird als zusätzlicher positiver Effekt für Diabetes-Patienten betrachtet.

### α-Amylase

Die Amylaseaktivitätsmessung unter dem Einfluss der Pflanzenextrakte wurde wie folgt durchgeführt:

Der Inkubationsmix für die Bestimmung der Amylaseaktivität besteht aus insgesamt 250 µL:
- 200 µL 50 mM/L MOPSO Puffer (pH 8,0) mit 1 % Gummi arabium
- 25 µL Pflanzenextrakt Lösung und
- 25 µL einer 0,35 mM/L α-Amylase Lösung

Diese Mischung wurde für 20 Minuten bei 38°C inkubiert. Die abschließliche Messung der pankreatischen Lipaseaktivität wird durchgeführt in einem Autoanalyser für chemische Chemie (Konelab, Kone).

Prinzip der chemischen Reaktion:
1. Ethylen-p-Nitrophenol-(Glucose)₇ → Ethylen-(Glucose)₃₋₄ + ρ-Nitrophenol-(Glucose)₂₋₄
2. p-Nitrophenol-(Glucose)-Reste → ρ-Nitrophenol (λ= 405 nm) + Glucose

Das Pipettenverfahren im Autoanalyser zur Bestimmung der α-Amylaseaktivitäten wurde wie folgt durchgeführt:
1. 120 µL Reagenz: Substrat + α-Glucosidase
2. 300 Sekunden Inkubation
3. 3 µL Testlösung
4. 180 Sekunden Inkubation

Die α-Amylaseaktivität wird bestimmt durch Messung von 5 Punkten in 120 Sekunden.

### Alkalische Phosphatase

Die Alkalische-Phosphatase-Aktivitätsmessung unter dem Einfluss von Pflanzenextrakten wurde wie folgt durchgeführt:
Der Inkubationsmix für die Bestimmung der Lipaseaktivität bestand aus
   - 200 µL einer 50 mM/L HEDTA Puffer (pH 8,0) enthaltend,
   - 25 µL Pflanzenextrakttestlösung und
   - 25 µL einer 0,014 mM/L alkalische Phosphatase Lösung
Die Mischung wurde inkubiert für 20 Minuten bei 22°C.
   Die abschließende Messung der pankreatischen Lipaseaktivität wurde durchgeführt in einem Autoanaylser für klinische Chemie (Konelab, Kone).
Reaktionsgleichung
   2-Amino-2-methyl-1-propanol + 4-Nitrophenylphosphatester → (2-Amino-2-methyl-1-Propanoö) Phosphat + 4-Nitrophenoxid (λ= 409 nm)
Das Pipettenverfahren im Autoanalyser zur Bestimmung der Aktivität der Alkalinen Phospatase war wie folgt:
   1. 150 µL Reagenz
   2. 300 sec. Inkubation
   3. 3µL Testlösung
   4. 120 sec. Inkubation

### Resultate

### % Enzymhemmung in enzymatischen Tests:

| | Sabalfrüchte |
|---|---|
| Lipase-methyl-resorufin (n) | 51 ± 12 (5) |
| Lipase-Triolein (n) | 87 (2) |
| α-Amylase | 2 |
| Alkaline Phospha-tase (n) | -11 ± 11 (5) |

Insbesondere in den ersten drei Messverfahren zeigt sich eine signifikante Enzymhemmung.

## Patentansprüche

1. Verwendung von Sabalfrüchten (Sabalae serulatae)
- als gesamte Frucht oder
- mit dem durch Pressen der Frucht hergestellten Öl als Einzelkomponente oder als Gemisch zur Herstellung eines oral zu applizierenden Arzneimittels, das als Lipase-Inhibitor bei der Behandlung oder der Risikoreduzierung von
- Diabetes oder
- dem metabolischen Syndrom oder
- anderen nachteiligen Effekten eines überhöhten Verzehrs von - Fetten - wirkt.

2. Verwendung von Sabalfrüchten *(Sabalae serulatae)*
- als gesamte Frucht oder
- mit dem durch Pressen der Frucht hergestellten Öl als Stoff, Stoffextrakt oder Stoffessenz entweder als Einzelkomponente oder als Gemisch zur Herstellung eines funktionellen Lebensmittels oder eines Wirkstoffes in Lebensmitteln mit Lipase inhibierender Wirkung bei der Behandlung oder bei der Risikoreduzierung von
- Diabetes oder
- dem metabolischen Syndrom oder
- anderen nachteiligen Effekten eines überhöhten Verzehrs von Fetten

## Claims

1. Use of sabal fruit *(Sabalae serrulatae)*
- as whole fruit or
- with the oil produced by pressing the fruit as an individual component or as a mixture for producing an orally administerable medication, which acts as a lipase inhibitor in the treatment or risk reduction of
- diabetes or
- metabolic syndrome or
- other disadvantageous effects of the excessive consumption of fats.

2. Use of sabal fruit *(Sabalae serrulatae)*
- as whole fruit or
- with the oil produced by pressing the fruit as a substance, substance extract or substance essence, either as an individual component or as a mixture for producing a functional food or an active substance in foods with a lipase-inhibiting effect in the treatment or risk reduction of
- diabetes or
- metabolic syndrome or
- other disadvantageous effects of the excessive consumption of fats.

## Revendications

1. Utilisation de fruits sabal (Sabalae serulatae)
■ sous forme de fruit entier ou
■ avec l'huile fabriquée en pressant le fruit
en tant que composant unique ou en tant que mélange destiné à fabriquer un médicament devant être appliqué oralement, qui agit en tant qu'inhibiteur de lipase lors du traitement ou de la réduction de risque du
■ diabète ou
■ du syndrome métabolique ou
■ d'autres effets désavantageux d'une consommation exagérée de graisses.

2. Utilisation de fruits sabal (Sabalae serulatae)
■ sous forme de fruit entier ou
■ avec l'huile fabriquée en pressant le fruit
en tant que substance, extrait de substance ou essence de substance, soit en tant que composant unique ou en tant que mélange destiné à fabriquer un aliment fonctionnel ou un agent actif dans les aliments ayant un effet inhibant la lipase lors du traitement ou de la réduction de risque du
■ diabète ou dru syndrome métabolique ou
■ d'autres effets désavantageux d'une consommation exagérée
■ de-graisses.
